# EUROPEAN PATENT APPLICATION

(11) **EP 1 683 488 A1**
(43) Date of publication of application: **26.07.2006**
(21) Application number: 05001117.0
(22) Date of filing: 20.01.2005
(51) Int. Cl.: A61B 17/02

(54) **Improved clamp, in special for surgical use**

(71) Applicant: ANSABERE SURGICAL S.L., 31110 Noain (Navarra) (ES)
(72) Inventor: Isturiz, Jesus Maria Insausti, 31110 Noain(Navarra) (ES)
(74) Representative: Isern-Jara, Nuria

(57) **Abstract**

An improved clamp, in special for surgical use, comprising fastening means and pressure means for attachment and stabilization of the clamp and the parts to be attached, and it is characterised in that said fastening means comprise a gripping jaw provided with two pieces, being disposed between them a washer (3), defining two portions (11) and (12) for arrangement of parts to attach between each pieces (1), (2) of the gripping jaw and the washer (3), being provided an perpendicular axle (4) that is screwed to lower piece (1) of gripping jaw and in its upper portion is provided with a bore for receiving a handle-like bar (6), so that the two portions (11), (12) can regulate their height to attach and immobilize the parts to be attached.

## Description

### DESCRIPTIVE MEMORY

### OBJECT OF THE INVENTION

The object of the present invention is to provide an improved clamp incorporating appreciable features and advantages with respect to other clamps known, which have the same purpose.

More specifically, it relates to a clamp for surgical use that comprises fastening means and pressure means for attachment and stabilization of the clamp and parts to be attached.

### BACKGROUNDS OF THE INVENTION

Currently, it is well known in the technical field of surgery the use of clamps for assembly of retractors or valves in bars comprising an appropriate frame hinged or fixed in order to facilitate surgical operations.

Said clamps used have complex configurations which can hinder and slow down the steps of assembly and removal of said clamps. Furthermore, this kind of clamps does not allow attachment of bars with different diameters, so that its use and application are shortened.

On the other hand, another drawback no less remarkable is in the fact that known clamps do not allow assembly for new retractors and valves between two clamps positioned, so as to be need to get back to handle the assembly of retractors and/or valves which are arranged as well as the frame hinged or fixed attached.

### SUMMARY OF THE INVENTION

The present invention has been developed in order to provide an improved clamp which overcome the aforesaid drawbacks, while presenting other additional advantages, which will become apparent from description, which is accompanied then.

The improved clamp of the present invention, in special for surgical use, belongs to the kind which comprises fastening means and pressure means for attachment and stabilization of the clamp and parts to be attached, and it is characterised in that said fastening means comprise a gripping jaw provided with two pieces, an upper piece and a lower piece, being disposed between them a washer, defining two portions and for arrangement of parts to attach between each pieces of gripping jaw and the washer, being provided an perpendicular axle that is screwed to lower piece of gripping jaw and in its upper portion is provided with a bore for receiving a handle-like bar and wherein said pressure means comprise at least a pair of springs disposed therein upper piece of gripping jaw, so that the two portions can regulate their height to attach and immobilize the parts to be attached.

Due to these features, a simple configuration clamp for surgical use is obtained, allowing a quick assembly and removal for the parts to be attached, such as bars having different diameters, so that time for carrying out said operations is reduced.

Furthermore, the clamp herein described has a maximum security for attachment of bars, which support the valves, and provides a greater working area and higher visibility for the surgeon.

Assembly and removal of bars which support valves can be made simply by means of a single hand by rotation of handle-like bar, aspect that simplify the operation with respect to known clamps and all of the components can be removed, allowing easy cleaning of each components that the clamp comprises.

According to another aspect of the clamp in accordance with the invention, is that the handle-like bar comprises abutment means in one end, which have an outer dimension portion higher than the bar, and removal means in opposite end, comprising a housing in the end of the bar that has a spring and a spherical body.

Further features and advantages of the improved clamp object of the present invention will become apparent from description of a preferred embodiment, illustrated only by way of non-limitative example in drawings which are accompanied, in which:

### BRIEF DESCRIPTION OF THE DRAWINGS

Figure 1 depicts a perspective view of an improved clamp assembled in accordance with the present invention;
Figure 2 depicts a perspective view of the clamp show in above figure from another point of view;
Figure 3 depicts a sectioned side elevational view of the clamp of the invention taken along the line A-A shown in figure 4;
Figure 4 depicts a top plan view of the improved clamp of the invention; and
Figure 5 depicts a sectioned elevational view taken along the line A-A and a side view of the handle-like bar.

### DESCRIPTION OF A PREFERRED EMBODIMENT

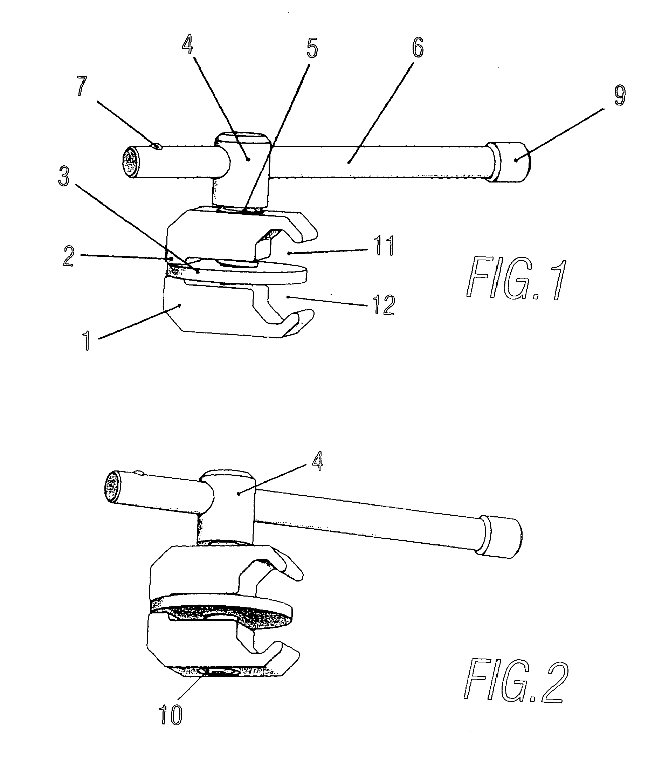

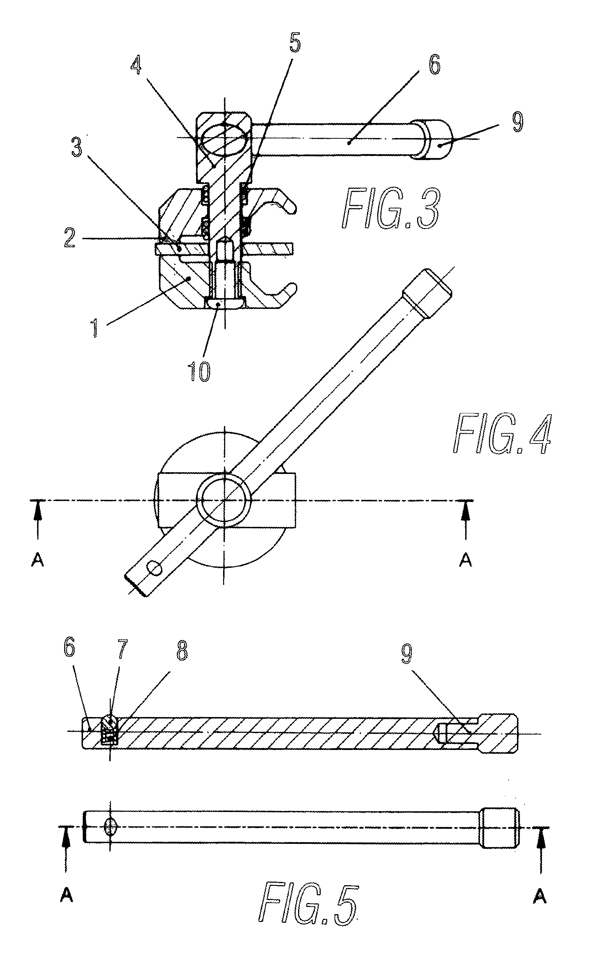

As shown in figures 1, 2 and 3, the clamp of the present invention which has been invented for arrangement and fixation of bars which support valves from surgery field has fastening means that comprise a gripping jaw provided with two pieces, an upper piece (2) and a lower piece (1), between them a washer is disposed, defining two portions (11) and (12) for arrangement of parts to be attached (not shown in drawings) among each pieces (1), (2) and washer (3), being provided an perpendicular metallic axle (4) that is screwed to lower piece (1) of gripping jaw and in its upper portion is provided with a bore for receiving a handle-like bar (6).

As appreciated, a screw screwed into lower axle end as fixing element for lower piece is provided.

In addition, said clamp has pressure means that comprise a pair of springs (5) disposed therein upper piece (2) of gripping jaw, so that the two portions (11), (12) can regulate their height to attach and immobilize the parts to be attached.

As shown in figure 5 in more detail, the handle-like bar (6) includes removal means which comprise a spring (8) and a spherical body (7), and abutment means (9), so that removal of bar (6) is very easy and it provides a greater working area for the surgeon.

Details, shapes, dimensions and further elements, as well as materials employed in the practical embodiment of the improved clamp of the invention will be able to replace by others to be technically equivalent and they do not depart from both principle of the invention and the scope defined by following claims which are included.

## Claims

1. Improved clamp, in special for surgical use, comprising fastening means and pressure means for attachment and stabilization of the clamp and the parts to be attached, **characterised in that** said fastening means comprise a gripping jaw provided with two pieces, an upper piece (2) and a lower piece (1), being disposed between them a washer (3), defining two portions (11) and (12) for arrangement of parts to attach between each pieces (1), (2) of the gripping jaw and the washer (3), being provided an perpendicular axle (4) that is screwed to lower piece (1) of gripping jaw and in its upper portion is provided with a bore for receiving a handle-like bar (6) and wherein said pressure means comprise at least a pair of springs (5) disposed therein upper piece (2) of gripping jaw, so that the two portions (11), (12) can regulate their height to attach and immobilize the parts to be attached.

2. Improved clamp as claimed in claim 1, **characterised in that** the handle-like bar (6) comprises abutment means (9) in one end, which comprise a outer dimension portion higher than the bar (6), and removal means in opposite end comprising a housing in the end of the bar (6) that has a spring (8) and a spherical body (7).
